# EUROPEAN PATENT SPECIFICATION

(11) **EP 3 020 335 B1**
(45) Date of publication and mention of the grant of the patent: **24.07.2019**
(21) Application number: 15194084.8
(22) Date of filing: 11.11.2015
(51) Int. Cl.: A61B 5/11, A61B 5/00, A61B 5/117, G01G 19/50, A61B 5/0205

(54) **METHOD AND APPARATUS FOR ADVISING PHYSICAL CONDITION AND RECORDING MEDIUM USING THE METHOD**
VERFAHREN UND VORRICHTUNG ZUR MITTEILUNG EINES KÖRPERLICHEN ZUSTANDES UND AUFZEICHNUNGSMEDIUM UNTER VERWENDUNG DES VERFAHRENS
PROCÉDÉ ET APPAREIL POUR DÉTERMINER LA CONDITION PHYSIQUE ET SUPPORT D'ENREGISTREMENT UTILISANT CE PROCÉDÉ

(30) Priority: 11.11.2014 US 201462077927 P; 29.01.2015 US 201562109059 P
(43) Date of publication of application: 18.05.2016
(73) Proprietor: HTC Corporation, Taoyuan City 330 (TW)
(72) Inventor: Lee, Yu-Cheng, Taoyuan District, Taoyuan City 330 (TW); Yeh, Chung-Ying, Taoyuan District, Taoyuan City 330 (TW)
(74) Representative: Carstens, Dirk Wilhelm

(56) References cited:
- EP-A1- 2 210 557
- US-A1- 2005 171 451
- US-A1- 2010 224 420
- US-A1- 2012 302 843
- US-A1- 2014 164 611

## Description

### BACKGROUND

To prevent from disease, modern people care more about their health. To trace the physical condition, people purchase various measuring equipments such as scale, thermometer, blood pressure meter, blood oxygen meter, blood-glucose meter, body fat monitor, heart rate monitor, pedometer, or electrocardiogram (ECG/EKG) for physiological measurements.

However, data measured by those equipments is individual to each other and is not well integrated to reveal overall physical condition of the user and to give proper advice for the user. For example, a scale can be used for measuring the weight, but the information brought by the weight is subjective and can only give a signal that a user is overweighed if the measurement is too high. However, based on the measurement, the user can only know that he has to lose weight, but usually has no idea about how to do it. As a result, the user may neglect the root cause of the high weight and simply go on a diet or do exercise, which may not be the best treatment for present physical condition.

US 2014/164611 A1 discloses methods, devices, and computer programs for creating a unified data stream from multiple data streams acquired from multiple devices. One method includes an operation for receiving activity data streams from the devices, each activity data stream being associated with physical activity data of a user. Further, the method includes an operation for assembling the unified activity data stream for a period of time. The unified activity data stream includes data segments from the data streams of at least two devices, and the data segments are organized time-wise over the period of time.

EP 2 210 557 A1 discloses an electronic device for estimating energy consumption of a person. The electronic device uses a mathematical model based on acceleration data for estimating the person's energy consumption as a function of the actual performed activity and acceleration values. The acceleration values are converted to an estimate of energy consumption of a given activity by scaling a value of time integrated acceleration data with an activity scaling parameter. The activity scaling parameters for different activities have been determined off-line by comparing measured energy consumptions over e.g. a day with estimated energy consumptions over the same period, where the estimated values are determined using the mathematical model.

US 2012/302843 A1 discloses a health management support system including: a unit that receives two or more types of physical information measured by a user along with measurement time data; an analyzing unit for analyzing the relationship between the received two or more types of physical information in accordance with a predetermined rule; an advice generating unit that generates advice based on a result of the analysis; and an advice output unit that outputs the generated advice. The analyzing unit has a knowledge file that stores the predetermined rule, and an engine unit for executing the analysis. The advice generating unit generates the advice for notifying the user of a goal achievement level by analyzing the two or more types of physical information measured in a first predetermined period.

US 2005/171451 A1 discloses a system and method for managing growth and development of a user, e.g., a child, wherein the system includes a biological information measuring module for acquiring at least two biological signals to be used for analyzing growth and development of the user, and for identifying the user by analyzing at least one biological signal of the at least two acquired biological signals and a biological information processing module for evaluating a development state and a growth level of the user according to the biological signals, for storing and managing personal data and the results of the evaluation of the user.

US 2010/224420 A1 discloses an information processing apparatus which processes an input signal from a load controller including an input surface and a load detecting means for detecting a load value applied to the input surface. The information processing apparatus detects a center-of-gravity position of a load applied to the input surface of the load controller based on the input signal from the load detecting means. The information processing apparatus determines whether or not the load value applied to the load controller is smaller than a predetermined value, based on the load value detected by the load detecting means, and when the result of determination is positive, executes a menu operation process based on the center-of-gravity position.

### SUMMARY

The disclosure provides a method and an apparatus for advising physical condition and a recording medium using the method, through which an advice most appropriate for a current physical condition of a user can be concluded and prompted for the user.

The present invention provides a method for advising physical condition as set forth in claim 1. In the method, multiple sensing devices are configured for a user to measure data related to the physical condition of the user. The data measured by the sensing devices is collected and integrated to estimate a current physical condition of the user. An advice is concluded at least based on the current physical condition of the user, and prompted for the user.

In an example of the present disclosure, after the step of collecting data measured by each of the sensing devices, the method further comprises uploading the collected data to a server comprising data collected from the other users to compare the collected data to the data from the other users according to a user information of the user and conclude the advice according to the collected data from the other users and the user information of the user, and receiving the advice from the server for prompting.

In an example of the present disclosure, the step of integrating the collected data to estimate the current physical condition of the user comprises comparing the collected data with physiological data recorded in a history of the user, and estimating the current physical condition according to a comparison result of the collected data and the physiological data.

In an example of the present disclosure, after the step of collecting the data measured by the sensing devices, the method further comprises determining an exercise activity currently exercised by the user based on the collected data, and concluding an exercise advice based on the estimated current physical condition and the determined exercise activity of the user.

In an example of the present disclosure, wherein the step of concluding the advice at least based on the current physical condition of the user further comprises concluding the advice based on an environmental data around the user.

In an example of the present disclosure, the sensing devices comprise a scale and the step of configuring the plurality of sensing devices for the user to measure the data of the user comprises receiving a plurality of user information, wherein each user information including an electrocardiogram (ECG) of a corresponding user, detecting a first user steps on the scale, measuring the ECG of the first user by using electrodes disposed in the scale in response to detecting the first user steps on the scale, comparing the measured ECG of the first user with the received user information and recognizing an identification of the first user at least based on a plurality of features analyzed from the ECG, and activating the scale to measure data of the first user in response to the recognized identification.

In an example of the present disclosure, the step of measuring data of the first user comprises determining whether the data of first user measured by the scale comprises a body fat percentage data according to the received user information, and measuring the body fat percentage data of the first user in response to determining the date of the first user comprises the body fat percentage data.

In an example of the present disclosure, the steps of integrating the collected data to estimate the current physical condition of the user and concluding the advice comprise weighting the collected data according to an exercise activity type of the user, wherein the exercise activity type is determined based on a plurality of data previously collected from the sensing devices and recorded in a history of the user, and estimating a total daily energy expenditure (TDEE) data of the user based on the weighted data and concluding the advice based on the TDEE data.

According to the present invention, the step of configuring the plurality of sensing devices for the user to measure the data of the user comprises detecting a load acted on a scale, determining the load is provided by a single object or two objects according to a shift on a center of gravity of the load and a change on a weight measured by the scale, determining whether the load lasts over a predetermined time in response to the load being determined as provided by the single object, entering a menu mode of the scale in response to the load lasting within the predetermined time, and keeping measuring the weight of the user in response to the load being determined as provided by two objects or the load lasting over the predetermined time.

The present invention provides an apparatus for advising physical condition, as set forth in claim 8. The connecting device is configured to connect with a plurality of sensing devices worn by or configured for a user, in which the sensing devices are configured to measure data related to the physical condition of the user. The storage device is configured to record a plurality of modules. The processor is coupled to the connecting device, the storage device and the prompt device, and configured to access and execute the modules recorded in the storage device. The modules include a data collecting module, an estimating module, an advice concluding module, and a prompt module. The data collecting module is configured to collect the data related to the physical condition measured by the sensing devices. The estimating module is configured to integrate the collected data to estimate a current physical condition of the user. The advice concluding module is configured to conclude an advice at least based on the current physical condition of the user. The prompt module is configured to prompt the advice by the prompt device.

In an example of the present disclosure, the apparatus further includes a communication device, which is configured to connect with a network, and the modules further comprise a communication module for uploading the collected data to a server comprising data collected from the other users through the network to integrate the collected data of the user, compare the collected data to the data from the other users according to a user information of the user, and conclude the advice according to the collected data from the other users and the user information of the user, and receiving the advice from the server.

In an example of the present disclosure, the estimating module compares the collected data with physiological data recorded in a history of the user, and estimates the current physical condition according to a comparison result of the collected data and the physiological data.

In an example of the present disclosure, the estimating module further determines an exercise activity currently exercised by the user based on the collected data, and the advice concluding module further concludes an exercise advice based on the current physical condition estimated and the determined exercise activity of the user.

In an example of the present disclosure, the advice concluding module concludes the advice based on an environmental data around the user among the collected data.

In an example of the present disclosure, the first sensing devices comprise a scale and the modules further comprise an identification recognizing module, which is configured to receive a plurality user information, wherein each user information including an ECG of each user, measure the ECG of a first user by using electrodes disposed in the scale in response to detecting the first user steps on the scale, compare the measured ECG of the first user with the received user information and recognizing an identification of the first user at least based on a plurality of features analyzed from the ECG, and activate the scale to measure data of the first user in response to the recognized identification.

In an example of the present disclosure, wherein the identification recognizing module further determining whether the data of first user measured by the scale comprises body fat percentage data according to the received user information, and the modules further include a calculation module, which is configured to measure the body fat percentage data of the first user in response to determining the date of the first user measured by the scale comprises the body fat percentage data.

In an example of the present disclosure, the estimating module further weights the collected data according to an exercise activity type of the user and estimates a TDEE data of the user based on the weighted data, wherein the exercise activity type is determined based on a plurality of data previously collected from the sensing devices and recorded in a history of the user, and the advice concluding module further concludes the advice based on the TDEE data.

According to the present invention, the modules further include an object detection module, which is configured to detect a load acted on a scale, determine whether the load is provided by a single object or two objects according to a shift on a center of gravity of the load and a change on a weight measured by the scale, determining whether the load lasts over a predetermined time in response to the load being determined as provided by the single object, enter a menu mode of the scale in response to the load lasting within the predetermined time, and keep measuring the weight of the user in response to the load being determined as provided by two objects or the load lasting over the predetermined time.

In an example of the present disclosure, wherein the sensing devices comprise an acceleration sensor, a gravity sensor (G sensor), a gyro sensor, a digital compass, a pedometer, a scale, a body thermometer, an ear thermometer, a blood pressure meter, a blood-glucose meter, a body fat monitor, a heart rate monitor, a hydration sensor, a lactate sensor, an electrocardiogram (ECG/EKG) sensor, a photoplethysmography (PPG) sensor, an air temperature gauge, a barometer, a hygrometer, an aerosol mass monitor, a gas sensor, or a combination thereof.

The disclosure provides a computer-readable recording medium configured for recording a program, loading the program via an electronic apparatus, and executing following steps. First, multiple sensing devices are configured for a user to measure data related to the physical condition of the user. The data measured by the sensing devices is collected and integrated to estimate a current physical condition of the user. An advice is concluded at least based on the current physical condition of the user, and prompted for the user.

In view of the above, in the method and apparatus for advising physical condition and the recording medium using the method, multiple sensing devices are configured to measure posture data and/or physiological data of the user, and/or environmental data around the user. The data is integrated to estimate a current physical condition of the user and conclude an advice most appropriate for the current physical condition of the user. The advice is then prompted for the user through visual or auditory messages so as to assist the user in managing his/her health condition.

In order to make the aforementioned and other features and advantages of the disclosure more comprehensible, several embodiments accompanied with figures are described in detail below.

### BRIEF DESCRIPTION OF THE DRAWINGS

The accompanying drawings are included to provide a further understanding of the disclosure, and are incorporated in and constitute a part of this specification. The drawings illustrate embodiments of the disclosure and, together with the description, serve to explain the principles of the disclosure.
FIG. 1 is a block diagram of an electronic apparatus according to an example of the disclosure.
FIG. 2 is a flow chart illustrating an advising method for a physical condition according to an example of the disclosure.
FIG. 3 is a block diagram of an electronic apparatus according to an example of the disclosure.
FIG. 4 is a flow chart illustrating an advising method for a physical condition according to an example of the disclosure.
FIG. 5 is a flow chart illustrating an advising method for a physical condition according to an example of the disclosure.
FIG. 6 is a flow chart illustrating an advising method for a physical condition according to an example of the disclosure.
FIG. 7 is a flow chart illustrating a method for measuring physiological data of the user according to an example of the disclosure.
FIG. 8 is a flow chart illustrating a method for activating a scale according to an example of the disclosure.

### DETAILED DESCRIPTION OF DISCLOSED EXAMPLES

In the disclosure, a method and an electronic apparatus for advising physical condition and a recording medium using the method are provided. The data related to the physical condition of the user, including physiological data, exercise activity data, and/or posture data of a user and/or environmental data around the user, respectively measured by a plurality of sensing devices are collected and integrated to conclude an advice most appropriate for a current physical condition and/or a current activity of the user. The integration may be implemented either in user's apparatus or in a cloud server based on an algorithm developed from big data previously collected from a mass of people. In another example, the algorithm may be a preset algorithm provided by a company, a gym, an expert, a trainer, a user, etc. and can be pre-stored or downloaded to user's apparatus and may be updated by the cloud server or provided by a cloud server. Examples are respectively given below for further illustration.

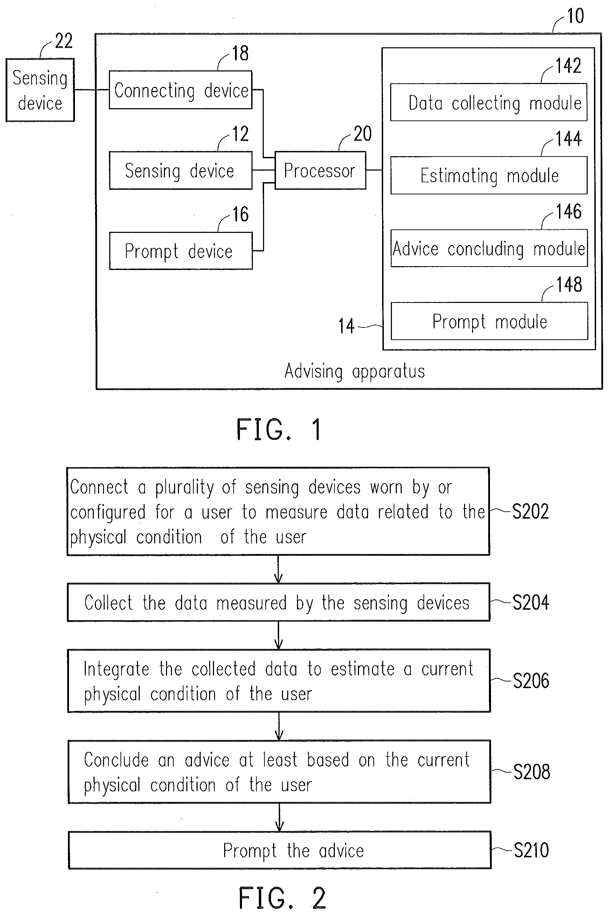

FIG. 1 is a block diagram of an electronic apparatus according to an example of the disclosure. Referring to FIG. 1, an electronic apparatus 10 of the present example is connected with a plurality of sensing devices, including external sensing devices and/or internal sensing devices. In the present example, the electronic apparatus 10 includes at least one sensing device 12 and is connected with at least one sensing device 22 by using a connecting device 18 or includes at least two different sensing devices 22 by using a connecting device 18. The electronic apparatus 10 further include a storage device 14, a prompt device 16, and a processor 20. The electronic apparatus 10 is, for example, a portable apparatus such as a smart phone, a cell phone, a tablet PC, a notebook computer, a wristband, a watch, a body weight scale, or a body fat percentage scale, but is not limited thereto.

The sensing device 12 may be a posture sensor such as, an acceleration sensor, a gravity sensor (G sensor), a gyro sensor, a digital compass, a pedometer, a posture sensor, or a combination of the above sensors for detecting acceleration, an inclination angle, a rotation angle or a facing direction of the electronic apparatus 10, and/or any other sensor which can alone or in combination detect the posture and the motion of the user of the electronic apparatus 10, but is not limited thereto.

The sensing device 12 may also be a physiological sensor such as a scale, a body thermometer, an ear thermometer, a blood pressure meter, a blood-glucose meter, a blood oxygen meter, a body fat monitor, a heart rate monitor, a hydration sensor, a lactate sensor, an electrocardiogram (ECG/EKG) sensor, a photoplethysmography (PPG) sensor, and/or any other sensor which can alone or in combination detect the physiological condition of the user, but is not limited thereto.

The sensing device 12 may also be an environmental sensor such as an air temperature gauge, a barometer, a hygrometer, an aerosol mass monitor, a gas sensor for detecting gases such as O₂, CO, CO₂, O₃, N₂, NO, NO₂, or CH₃OH, an alcohol sensor, and/or any other sensor which can alone or in combination detect the environmental condition, but is not limited thereto.

The sensing device 22 may be a wearable device implemented in a form of a sticker, a wristband, a heart rate band, a glass, an earphone, a helmet, a mask, a necklace, a watch, a ring, a bracelet, a clothes, a belt, or a shoe, etc., capable of being worn by a user, or an instrument configured for measuring data of the user.

Similar to the sensing device 12, the sensing device 22 may be, for example, a posture sensor, a motion sensor, a physiological sensor, or an environmental sensor. Types and functions of those sensors are similar to those of the sensing device 12 described in the above, thus the description is omitted herein.

The sensing devices 22 may be connected with the electronic apparatus 10 through the connecting device 18 in a wired or a wireless manner. As for the wired manner, the connecting device 18 may be a universal serial bus (USB), a RS232, a universal asynchronous receiver-transmitter (UART), an inter-integrated circuit (I²C), a serial peripheral interface (SPI), a display port, a thunderbolt, or a local area network (LAN) interface. As for the wireless manner, the connecting device 18 may be a wireless fidelity (Wi-Fi) module, a Bluetooth module, an infrared rays (IR) module, a near-field communication (NFC) module, or a device-to-device (D2D) module.

The storage device 14 may be any type of a random access memory (RAM), a read-only memory (ROM), a flash memory, a hard disk, or a similar device or a combination of the above devices. In the present example, the storage device 14 is configured to record the data collected from the sensing device 22 and/or the sensing device 12. Further, the storage device 14 may store a database including mass physiological data in different regions, culture, genders, ages, job types, sexes, and races, etc. The storage device 14 may also record a program capable of executing an algorithm for integrating the collected data, estimating a physical condition of the user, and providing an advice most suitable for the estimated physical condition. The program comprises a data collecting module 142, an estimating module 144, an advice concluding module 146, and a prompt module 148.

The prompt device 16 is, for example, a display, a speaker, a LED array, or a vibrator, or a combination thereof, capable of prompting visual, auditory, and/or vibration messages which indicate the advice provided by the processor 20. In another example, the sensing deice 22 may also have a prompt device (not shown) similar to the prompt device 16 for prompting the advice provided by the processor 20 to the user. For example, an earphone, a wristband, a glass, a helmet, a watch, a body weight scale, a body fat scale, etc. may also have a display and/or a speaker to provide the advice to the user.

The processor 20 is coupled to the sensing device 12, the storage device 14, the prompt device 16, and the connecting device 18. The processor 20 may be a central processing unit (CPU), or a programmable general purpose or special purpose microprocessor, a digital signal processor (DSP), a programmable controller, an application specific integrated circuit (ASIC) or any other similar device or a combination of the above devices. In the present example, the processor 20 is configured to load the data collecting module 142, the estimating module 144, the advice concluding module 146, and the prompt module 148 recorded in the storage device 14 for executing the advising method for the physical condition according to the examples of the disclosure.

FIG. 2 is a flow chart illustrating an advising method for a physical condition according to an example of the disclosure. Referring to FIG. 1 and FIG. 2, the method of the present example is applicable to the electronic apparatus 10 in FIG. 1. In the following, the method of the present example is described in detail with reference to the devices of the electronic apparatus 10 in FIG. 1.

First, the processor 20 connects the sensing device 22 worn by or configured for a user to measure data of the user (step S202). In one example, the processor 20 may detect the sensing device 22 and connect with the detected sensing device 22 by using the connecting device 18. The sensing device 22 may be worn by a user or configured for the user and used to measure data of the user. The measurement of the sensing device 22 may be triggered either by the processor 20 or by the sensing device 22 itself, which is not limited herein.

Next, the processor 20 executes the data collecting module 142 to collect the data measured by the sensing device 22 and the sensing device 12 (step S204), and then executes the estimating module 144 to integrate the collected data to estimate a current physical condition of the user (step S206). In one example, the processor 20 may inquire a database recorded in the storage device 14 or in a cloud server to search for the physiological data that is matched with the collected data, so as to estimate the physical condition of the user being measured.

Then, the processor 20 executes the advice concluding module 146 to conclude an advice at least based on the current physical condition of the user (step S208). In detail, if the current physical condition, for example according to the sleeping quality track, resting heart rate measurement, etc., shows the user is tired, the processor 20 may conclude the advice such as slowing down the pace, reducing strength of exercise, reducing a time of exercise, or ceasing doing exercise. If the current physical condition shows the user is still energetic, the processor 20 may conclude the advice such as speeding up, increasing strength or amount of exercise, or prolonging a time of exercise.

Finally, the processor 20 executes the prompt module 148 to prompt the advice through the prompt device 16 (step S210). The advice may be prompted in a form of at least a visual message, at least an audio message and/or vibration signals capable of notifying the user of the actions most appropriate for his/her current physical condition. In another example, the processor 20 may forward the advice to the sensing device 22 for being prompted on the sensing device 22.

For example, when the user is doing physical activity, the electronic apparatus 10 may become a voice coach to prompt advices so as to help the user during the physical activity. The advices may be asking the user to do more exercise according to the data collected by the sensing devices. In one example, the advices may be asking the user to do more exercise accompanied with a music automatically played according to the user's heart rate or the motion patterns of the sensing devices, so as to encourage the user to do the exercise. For example, when the sensing device 22 detects user's pace is slowing down, the electronic apparatus 10 may change the playlist to play more exciting music and encourage user to speed up or even use voice to remind user of speeding up for better performance. In another example, when the sensing device 22 detects user's heart rate is too fast, the electronic apparatus 10 may play music that is helpful for slowing down the heart rate and remind the user to speed down or reduce the amount of the exercise activity.

Based on the above, multiple kinds of data related to the physical condition of the user are integrated and accordingly a better health tip can be given to the user, so as to help the user to know what to do for current physical condition.

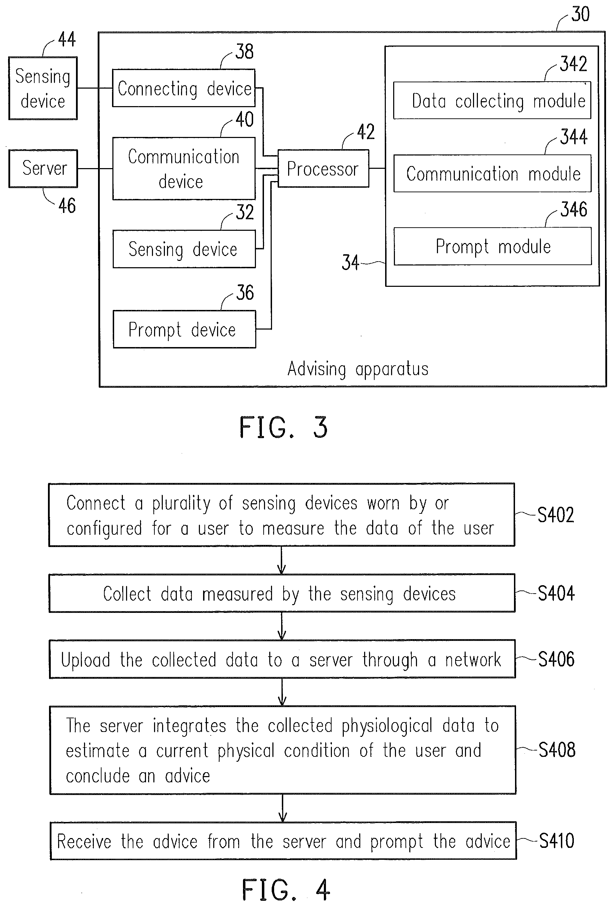

It is noted that, in the former example, the advice is generated on the electronic apparatus 10. In another example, the advice can be provided through Internet. In detail, FIG. 3 is a block diagram of an electronic apparatus according to an example of the disclosure. Referring to FIG. 3, an electronic apparatus 30 of the present example may be connected with a plurality of sensing devices, including external sensing devices and/or internal sensing devices. In the present example, the electronic apparatus 30 includes at least one sensing device 32 and is connected with at least one sensing device 44 by using a connecting device 38. The electronic apparatus 30 further includes a prompt device 36, a communication device 40, and a processor 42. The electronic apparatus 30 is, for example, a portable apparatus such as a smart phone, a cell phone, a tablet PC, a notebook computer, a wristband, a watch, a body weight scale, or a body fat percentage scale, but is not limited thereto. Types and functions of the sensing device 32, the prompt device 36, connecting device 38, and sensing device 42 are the same as or similar to those of the sensing device 12, the prompt device 16, connecting device 18, and sensing device 22, which are not repeated hereinafter.

Unlike the foregoing example, in the present example, the communication device 40 is disposed in the electronic apparatus 30 for communicating with a server 46 through a network so as to obtain the advice for the physical condition of the user. To be specific, the communication device 40 may support at least one of the following technologies in wireless signal transmission: Global System for Mobile Communication (GSM) system, Personal Handy-phone System (PHS), Code Division Multiple Access (CDMA) system, Wireless fidelity (Wi-Fi) system, Worldwide Interoperability for Microwave Access (WiMAX) system, Radio Repeater or Radio Broadcaster, but the present disclosure is not limited to the above.

Types and functions of the storage device 34 are the same as or similar to those of the storage device 14, but the storage device 34 records a program comprising a data collecting module 342, a communication module 344, and a prompt module 346. The processor 42 may load the data collecting module 342, the communication module 344, and the prompt module 346 recorded in the storage device 14 for executing the advising method for the physical condition according to the examples of the disclosure.

FIG. 4 is a flow chart illustrating an advising method for a physical condition according to an example of the disclosure. Referring to FIG. 3 and FIG. 4 together, the method of the present example is applicable to the electronic apparatus 30 in FIG. 3. In the following, the method of the present example is described in detail with reference to the devices of the electronic apparatus 30 in FIG. 3.

First, the processor 42 connects the sensing device 44 worn by or configured for a user to measure data of the user (step S402). Then, the processor 42 executes the data collecting module 342 to collect the data measured by the sensing device 32 and the sensing device 42 (step S404). The steps S402 and S404 are identical or similar to the steps S202 and S204 in the foregoing example, and therefore the detail is not repeated herein.

Different from the foregoing example, in the present example, the processor 42 executes the communication module 344 to upload the collected data to the server 46 through a network by using the communication device 40 (step S406), in which the server 46 may include a database recording mass physiological data in different regions, culture, genders, ages, job types, sexes, and races, etc. and/or the historical data of the user previously collected and recorded in the server 46.

Further, the server 46 may execute an algorithm to integrate the collected data related to the physical condition to estimate the current physical condition of the user and conclude an advice suitable for current physical condition of the user according to the collected mass physiological data in different regions, culture, genders, ages, job types, sexes, and races, etc. (step S408).

Finally, the processor 42 may receive the advice from the server 46 through the communication device 40 and accordingly executes the prompt module 346 to prompt the advice (step S410). The advice may be prompted in a form of at least a visual message, at least an audio message, and/or vibration signals capable of notifying the user of the actions most appropriate for his/her current physical condition.

It is noted that, in the present example, since the database is established in the server 46 and the algorithm is implemented in the server 46, the amount of data related to the physical condition recorded in the database may be accumulated as the time passed by so as to render the data related to the physical condition more objectively and accurately and more proper advices will be generated. Further, the algorithm being executed for integrating the physiological data may also be upgraded to fit different requirements such as regions, culture, genders, ages, job types, sexes, and races, etc.

Based on the above, physical condition data of the user can be integrated through a cloud server such that not only a calculation cost of the electronic apparatus can be reduced, but a better or up-to-date advice can be obtained in the benefit of upgradeable database and algorithm.

In another example, the currently collected data related to the physical condition is compared with historic data of the user, for example per day, per week, per month, and even per year, especially the recent data related to the physical condition, so as to provide an appropriate advice in accordance with a former condition of the user or a former activity exercised by the user.

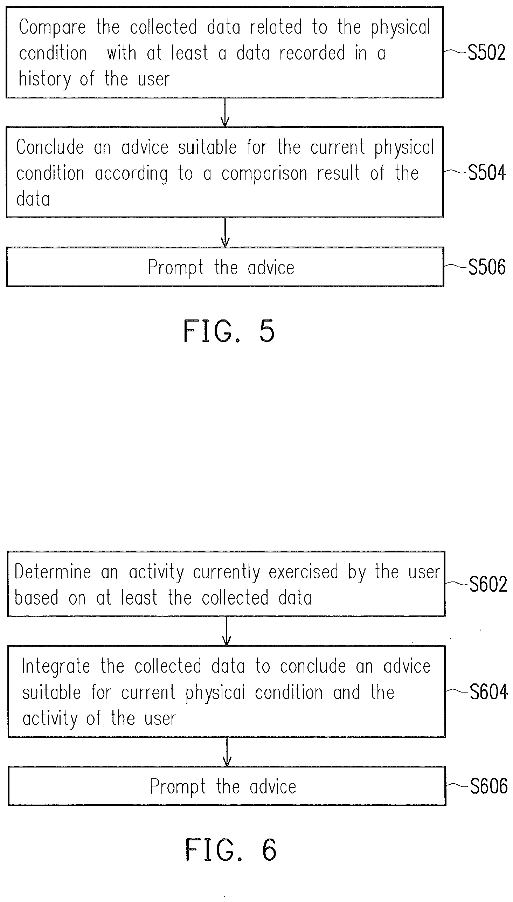

FIG. 5 is a flow chart illustrating an advising method for a physical condition according to an example of the disclosure. Referring to FIG. 1 and FIG. 5 together, the method of the present example is subsequent to the step S204 of FIG. 2.

In detail, after collecting the data, the processor 20 executes the estimating module 144 to compare the collected data related to the physical condition with the data recorded in a history of the user (step S502). The data being compared may be the data recorded within a predetermined period.

For example, a heart rate being measured and recorded before sleep may be retrieved and compared with the heart rate being measured right after the user gets up. The movements of the user detected and/or the physiological data of the user measured during the sleep may be further incorporated to determine the sleep quality of the user, which may also be used as a reference to determine the physical condition of the user and conclude the advice. For example, if user's resting heart rate before waking up is higher than the average of his/her previous record, for example, 5 beats higher. Based on this condition, the electronic device may advise user to reduce the strength or amont for the exercise or take one more day rest and not to do any exercise today due to the resting heart rate and/or sleeping quality indicating the physical body is still tired. In one example, the number of higher resting heart rate may be adjusted according to big data collected from mass users.

Then, the processor 20 executes the estimating module 144 to estimate the current physical condition according to a comparison result of the collected data and executes the advice concluding module 146 to conclude an advice suitable for the current physical condition (step S504), and finally the processor 20 executes the prompt module 148 to prompt the advice through the prompt device 16 (step S506). For example, the processor 20 may determine that the user is overtired according to a difference between the heart rates before and after the sleep and a number of sleep cycle detected during the sleep. In another example, the user will be determined overtired if the heart rates after the sleep is much higher than the average heart rate value or historical heart rate data of the user after sleep.

Based on the above, a former physical condition is considered based on the historic data and accordingly a better health tip complied with the change of the physical condition of the user can be given to the user.

In another example, the activity currently exercised by the user is further taken into consideration and the advice for the physical condition is adjusted to fit the current activity of the user in real time.

FIG. 6 is a flow chart illustrating an advising method for a physical condition according to an example of the disclosure. Referring to FIG. 1 and FIG. 6 together, the method of the present example is also subsequent to the step S204 of FIG. 2.

In detail, after collecting the data, the processor 20 executes the estimating module 144 to determine an activity currently exercised by the user based on at least the collected data (step S602). In one example, the processor 20 may determine that the user is doing weight training, jogging, swimming, cycling, walking, etc. if the processor 20 detects a plurality of regularly and repeatedly performed actions, for example the motion patterns detected by the sensing device, based on the collected posture data,. In another example, the processor 20 may determine that the user is doing yoga or Tai-Chi based on a blood oxygen level and/or a PPG waveform.

The processor 20 also executes the estimating module 144 to integrate the collected data to estimate a current physical condition of the user and then executes the advice concluding module 146 to conclude an advice based on the estimated current physical condition, including the determined activity, of the user (step S604). Finally, the processor 20 executes the prompt module 148 to prompt the advice through the prompt device 16 (step S606).

It is noted, to conclude the advice most appropriate for the current physical condition and activity of the user, in one example, the processor 20 may conclude the exercise advice based on the current physical condition and further based on the information input by the user. In another example, the processor 20 may conclude the advice for the exercise activity based on the collected posture data. In yet another example, the processor 20 may conclude the advice for the exercise activity based on the collected environmental data around user. Examples are given below for further illustration.

In one example, if a user sets up a goal like reducing a weight of 15 lbs or reducing a body fat percentage by 2%, the processor 20 may calculate a fitness plan according to the current weight and/or body weight percentage of the user. When the user wears a sports band/sensors, the processor 20 may determine that the user is walking based on the heart rate and/or the steps measured by the sports band. When the user wears the sports band/sensors and walks, the processor 20 may compare the walking steps or a walking distance measured by the sports band/sensors with the previously calculated fitness plan to determine whether the amount of walking is enough, and finally conclude an advice such as walking faster or walking further for a certain amount of time or distance. In another example, the user may wear one or more different sensors on different body positions, for example a wristband, a watch, a sensor on a shoe, a sensor on a foot, a heart rate band, etc. for determining the exercise activity based on the collected data and concluding an advice for the user. For example, what kind of weight training and the amount that the user doing the weight training may also be determined based on the collected data, for example the motion patterns, from the sensors on different body positions. In another example, whether the posture of the exercise activity, for example the posture of weight training, is correct can be determined according to the collected data, and an advice for the posture can be provided.

In one example, if the strength or the amount of the original fitness plan is not higher than the strength or amount of the user's regular physical activity or current activity based on the previous collected data, the processor 20 may conclude a new fitness plan for the user. In this example, the processor 20 may get the information of the strength or amount of the user's regular physical activity or current activity by receiving the information input by the user or based on the data received by the sensing devices.

In one example, if the collected data shows the user is tired and the collected posture data shows the steps of the user is heavy, the posture of the user is unbalanced, the foot is fatigue based on the sensors on shoes, etc. the processor 20 may advise user to adjust the posture, take a rest or stop to do exercise.

In one example, if the collected data, for example posture data, shows the user is doing weight training but the collected physiological data shows lactic acid accumulated in the user's muscle is high, the processor 20 may advise user to reduce the strength or amount of exercise, so as to avoid injury.

In one example, if the collected physiological data shows the body temperature, for example collected by an earphone with body temperature sensor, of the user is high or the body water of the user is low, and the collected environmental data shows current temperature or humidity is high, the processor 20 may determine that a risk to cause heatstroke is high and therefore advise user to drink water or move to a cooler place. In another example, if the environment sensor detects that the environment around the user is not suitable for take exercise, for example the air quality is unhealthy, the temperature is too high, or the humidity is too high, etc., on the way of the user taking exercise or commuting, and the processor 20 will advise the user not to take exercise or use another way to commute.

Based on the above, the activity currently exercised by the user is further estimated and an advice most appropriate for the current physical condition of the user, and/or the current activity of the user, and/or the current environment around the user can be given to the user.

It is noted that the electronic apparatus described in the above examples may also be the sensing device itself such as a scale for measuring body weight and/or body fat percentage, a blood pressure meter, or any other physiological data measuring sensor. The electronic apparatus may also communicate with other electronic apparatuses and share the physiological data measured thereby with those electronic apparatuses such that even though the electronic apparatus can only measure one kind of physiological data, it can still get other kinds of data related to the physical condition from other electronic apparatus and accordingly show all kinds of physiological data for the user and/or conclude the advice for the physical condition of the user. For example, when the user steps on a scale, the scale may not only show the weight measured thereby, but also shows weight management tips such as how much food the user should take, what kind and how much activity exercised before, how much calories will burn for keeping current activity, even the sleep quality of yesterday, etc. As a result, the user can have an overall picture of his/her physical condition and know the plans for weight management.

The apparatus illustrated by the block diagram of FIG. 1 may be considered as a tip system for measuring data related to the physical condition and providing advice for the physical condition. Considering the plurality of sensing devices are required for the tips system, the disclosure also provides a convenient method to help user to setup these sensing devices when using them for the first time.

For example, the sensing devices may include a wristband, a heart rate chest band, and a smart scale. The smart scale may link to a network individually for daily measurement tracking. However, configuring the smart scale to link to the network through, for example, a WiFi hotspot, may not be easy to do on the scale. The heart rate chest band can pair with the wristband and trace the heart rate of the user during physical activity, send the heart rate to the wristband for the user to check, and upload the heart rate to a server later. The wristband is also capable of pairing with a smart phone to upload all collected data through the smart phone to a server for further analysis. Since these sensing devices are capable of connecting with at least one other sensing device, the setup may become a burden when doing the setup for the first time.

To solve this problem, the sensing devices may be paired in advance in the factory before being sent to users. Meanwhile, a unique ID information, for example a Bluetooth mac address, of the heart rate chest band or the smart scale may be stored in a wristband first. A package of these devices may include a quick setting mechanism that can turn on the wristband and the smart scale at once when the user opens it for the first time. The user may download a software application to a smart phone and execute the software application to select pairing the package. When the user switches on the package, the smart scale and the wristband will be turned on at the same time. Then, the software application may allocate the wristband and pair with it directly. Since this wristband is a private secure device, the wristband may request the user to confirm the pairing. Once the user confirmed, the pairing is done and meanwhile the smart scale and the heart rate chest band may send the Bluetooth mac address to the smart phone. When the smart scale is turned on, the smart scale starts to scan nearby available WiFi hotspots to obtain a service set identification (SSID) list. Since the smart phone has acquired the Bluetooth mac address of the smart scale, the smart phone may pair with the smart scale immediately. The smart scale passes all the available SSIDs to the smart phone, and then the smart phone compares those SSIDs with a previously configured SSID to allocate the latest one and display an interface to ask the user to provide a password for configuring the WiFi hotspot on the smart scale. Moreover, the software application may pass the user's account information to the smart scale and remind the user to stand on the smart scale for measuring the weight. Meanwhile, the smart scale may detect the user's biometric identification so as to be activated to measure the weight of the user, link to the user's account, and update the measurement to the server.

In one example, the smart scale may include electrodes used to measure body fat by using bio-electrical impedance analysis (BIA). The electrodes may be further used to measure an electrocardiogram (ECG) of the user. In detail, since the ECG is unique to every people, the ECG can be used as an identification of a user for identifying the user, activating the scale, and measuring the physiological data of the user.

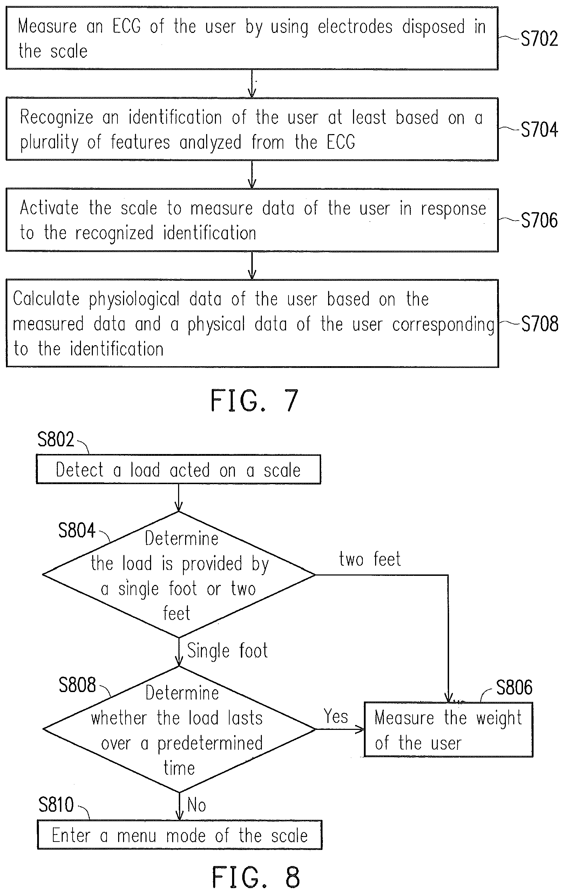

In detail, FIG. 7 is a flow chart illustrating a method for measuring physiological data of the user according to an example of the disclosure. Referring to FIG. 1 and FIG. 7 together, in the present example, the processor 20 of the electronic apparatus 10 may execute an identification recognizing module (not shown) stored in the storage device 14 for recognizing an identification of the user, and execute a calculation module (not shown) for calculating physiological data of the user, such as a body fat percentage of the user.

First, the processor 20 measures the ECG of the user by using electrodes disposed in the scale (step S702). It is noted that different from active detection of body fat that conducts a feeble constant current through a human body, the ECG is passively detected by the electrodes receiving electric currents transmitted from the heart without applying the current to the human body, and therefore ECG detection of the present example is safer for children, pregnant women, or people wearing a pacemaker.

Next, the processor 20 recognizes an identification of the user at least based on a plurality of features analyzed from the ECG (step S704). There are at least twelve to sixteen distinguishable features in the ECG that can be used as a reference to identify different users. The reference ECG data for identification of each user may be previous input or recorded and associated with the user information of each user when the scale receives the personal user information from the user's input, the other electronic devices, or cloud services.

In another example, in addition to the ECG, a fingerprint of a feet of the user detected by using a fingerprint sensor disposed on the scale or a voice of the user detected by using a microphone disposed on the scale may be used as an recognized identification of a user. Furthermore, such biometric features may be further incorporated with the ECG to recognize the identification of the user so as to increase the accuracy of recognition.

Then, the processor 20 activates the scale to measure data of the user in response to the recognized identification (step S706). In detail, the processor 20 may compare the recognized identification with a plurality of identifications previously set by the users so as to identify the user currently stands on the scale. In one embodiment, the processor 20 will determine whether the data of user measured by the scale includes body fat percentage data according to the user information corresponding to the user, and will measure the body fat percentage data of the user if the measured data of the user includes the body fat percentage data according to the user information.

Finally, the processor 20 calculates physiological data of the user based on the measured data and a physical data of the user corresponding to the identification (step S708). In detail, since the measuring and calculation of some physiological data (e.g. body fat percentage, body water percentage, body mass index (BMI), or basal metabolic rate (BMR), etc.) requires physical data of the user such as height, weight, age, and even amount of exercise or workload, the processor 20 may inquire the physical data of the user by using the identification of the user previously recognized so as to calculate the desired physiological data.

In one example, an exercise activity type of the user will be determined according to the collected exercise activity data of the user for one day, one week, or even one month. For example, the exercise activity types may have 5 groups including (i) sedentary (little or no exercise); (ii) lightly active (1-3 days per week); (iii) moderately active (3-5 days per week); (iv) very active (rigorous exercise 6-7days per week); and (v) extremely active (daily rigorous exercise and physical job). Furthermore, a total daily energy expenditure (TDEE) data of the user will be estimated based on the exercise activity type and BMR data of the user.

Through aforesaid method, the user stepping on the scale can be automatically identified every time the user uses the scale and appropriate physical data can be applied to calculate the physiological data without miscellaneous selections or inputs by the user.

According to the invention, intent of the user for using the scale may be determined so as to trigger the scale to enter a menu mode or do a normal physiological data, for example body weight and body fat percentage, measurement. In detail, FIG. 8 is a flow chart illustrating a method for activating a scale according to an example of the disclosure. Referring to FIG. 1 and FIG. 8, in the present example, the electronic apparatus 10 may be a scale. The processor 20 of the electronic apparatus 10 may execute an object detection module (not shown), for example a module including load cells, to detect a load acted on a scale (step S802) and determines the load is provided by a single foot or two feet according to a shift on a center of gravity of the load and a change on a weight measured by the scale (step S804). In detail, according to a regular steps for measuring the weight, the user always step on one foot first and then the other and the postures that the one or two feet step on are different, which causes a center of gravity of a load detected by the scale is moved. Specifically, the measurement of the load detected by the scale when the user steps on one foot may be approximately a half of the measurement of the load when the user steps on two feet. Further, the center of gravity is originally at a center of the scale, but moves to the left when the user steps on left foot at a left portion of the scale. The center of gravity may move back to the center when the user steps on right foot at a right portion of the scale. Based on aforesaid two conditions, it is easy to determine the load detected by the scale is provided by a single foot or two feet.

If it is determined the load is provided by two feet, the processor 20 may confirm that the user intends to measure the weight, and therefore measure the weight of the user (step S806). In one example, if it is determined the load is provided by only one foot, the processor 20 may further determines whether the load lasts over a predetermined time, for example 2 seconds (step S808). If the load lasts over the predetermined time, the processor 20 also will determine that the user intends to measure the weight, and therefore measure the weight of the user (step S806). If the load does not last over the predetermined time, the processor 20 will determine that the user intends to enter a menu mode of the scale (step S810). In one example, if the status of the scale is power-off, the scale will be automatically triggered after determining the user steps on the scale, and then the aforementioned determining process will be proceeded.

Through aforesaid method, the intent of the user for the scale can be determined and a corresponding function can be accurately executed without extra operations or inputs by the user.

The present application further provides a recording medium, which records a computer program for executing various steps of the advising physical condition method described above. The computer program is composed of a plurality of code snippets (for example, an organization chart establishment code snippet, a form approval code snippet, a settings code snippet, and a deployment code snippet). After these code snippets are loaded into an electronic apparatus and executed by the same, the steps of the advising physical condition method described above can be accomplished.

To sum up, the method and the apparatus for advising physical condition and a recording medium using the method of the present disclosure collect data related to the physical condition from one or multiple sensing devices, estimate a current physical condition and/or a current/recent activity of the user and/or environmental data around the user based on the collected physiological data, the historic data, the activity data, the environmental data, and conclude an advice most appropriate for the current physical condition and/or the current activity of the user through a previously established database and algorithm. As a result, the advice being prompted may be most useful to the user and help the user in managing his/her health condition.

## Claims

1. A method of using an electronic scale (10) for advising a physical condition, , the method comprising the following steps implemented by a processor (20) of the scale (10): configuring (S202, S402) a plurality of sensing devices (12, 22, 32, 44) for a user to measure the data related to the physical condition of the user;
collecting (S204. S404) the data measured by the sensing devices (12, 22, 32, 44);
integrating (S206, S408) the collected data to estimate a current physical condition of the user;
concluding (S208, S408) an advice at least based on the current physical condition of the user; and
prompting (S210, S410) the advice; **characterized in that** the step of configuring (S202, S402) comprises: detecting (S802) a load acted on the scale (10); determining (S804) whether the load is provided by a single object or two objects according to a shift on a center of gravity of the load and a change on a weight measured by the scale (10);
determining (S808) whether the load lasts over a predetermined time in response to the load being determined as provided by the single object; triggering the scale to enter (S810) a menu mode in response to the load lasting within the predetermined time; and triggering the scale to keep measuring (S806) the weight of the user in response to the load being determined as provided by two objects or the load lasting over the predetermined time.

2. The method according to claim 1, wherein after the step (S404) of collecting data measured by each of the sensing devices (32, 44), the method further comprises:
uploading (S406) the collected data to a server (46) comprising data collected from the other users to compare the collected data to the data from the other users according to a user information of the user and conclude the advice according to the collected data from the other users and the user information of the user; and
receiving (S410) the advice from the server (46) for prompting.

3. The method according to claim 1 or 2, wherein after the step (S204, S404) of collecting the data measured by the sensing devices, the method further comprises:
determining (S502) an exercise activity currently exercised by the user based on the collected data; and
concluding (S504) an exercise advice based on the estimated current physical condition and the determined exercise activity of the user.

4. The method according to any one of claims 1 to 3, wherein the step (S208, S408, S504) of concluding the advice at least based on the current physical condition of the user further comprises:
concluding the advice based on an environmental data around the user.

5. The method according to any one of claims 1 to 4, wherein the sensing devices (12, 22, 32, 44) comprise a scale and the step (S202, S402) of configuring the plurality of sensing devices (12, 22, 32, 44) for the user to measure the data of the user comprises:
receiving a plurality of user information, wherein each user information comprising a recognized identification information of a corresponding user, and wherein the recognized identification information comprising at least one biometric feature of the corresponding user;
detecting a first user steps on the scale;
in response to detecting the first user steps on the scale, measuring the at least one biometric feature of the first user by using a sensor for the at least one biometric feature in the scale;
comparing the measured at least one biometric feature of the first user with the received recognized identification information and recognizing an identification of the first user at least based on the at least one biometric feature;
determining whether the data of first user measured by the scale comprises a body fat percentage data according to the received user information; and
activating the scale to measure data of the first user in response to the recognized identification and measuring the body fat percentage data of the first user in response to determining the date of the first user comprises the body fat percentage data.

6. The method according to any one of claims 1 to 5, wherein the steps (S206, S408) of integrating the collected data to estimate the current physical condition of the user and concluding (S208, S408) the advice comprise:
weighting the collected data according to an exercise activity type of the user, wherein the exercise activity type is determined based on a plurality of data previously collected from the sensing devices and recorded in a history of the user; and
estimating a total daily energy expenditure, TDEE, data of the user based on the weighted data and concluding the advice based on the TDEE data.

7. A non-transitory computer readable medium comprising instructions to cause a scale to execute the method according to any one of claims 1 to 6.

8. A scale (10) for advising a physical condition, the scale (10) comprising:
a connecting device (18, 38), connecting with a plurality of sensing devices (22, 44) worn by or configured for a user, wherein the sensing devices (22, 44) are configured to measure data related to the physical condition of the user;
a prompt device (16, 36);
a storage device (14, 34), configured to record a plurality of modules (142, 144, 146, 148; 342, 344, 346); and
a processor (20, 42), coupled to the connecting device (18, 38), the storage device (14, 34) and the prompt device (16, 36), and configured to access and execute the modules (142, 144, 146, 148; 342, 344, 346) recorded in the storage device (14, 34), wherein the modules (142, 144, 146, 148; 342, 344, 346) comprise:
a data collecting module (142, 342), collecting the data related to the physical condition of the user measured by the sensing devices (22, 44);
an estimating module (144), integrating the collected data to estimate a current physical condition of the user;
an advice concluding module (146), concluding an advice at least based on the current physical condition of the user;
a prompt module (148), prompting the advice by the prompt device (16, 36); and **characterized by**
an object detection module, detecting a load acted on the scale (10), determining the load is provided by a single object or two objects according to a shift on a center of gravity of the load and a change on a weight measured by the scale (10), determining whether the load lasts over a predetermined time in response to the load being determined as provided by the single object, triggering the scale (10) to enter a menu mode in response to the load lasting within the predetermined time, and triggering the scale (10) to keep measuring the weight of the user in response to the load being determined as provided by two feet or the load lasting over the predetermined time.

9. The scale according to claim 8, further comprising:
a communication device (40), configured to connect with a network, and the modules further comprise a communication module (344) for uploading the collected data to a server (46) comprising data collected from the other users through the network to integrate the collected data of the user, compare the collected data to the data from the other users according to a user information of the user, and conclude the advice according to the collected data from the other users and the user information of the user, and receiving the advice from the server (46).

10. The scale according to claim 8 or 9, wherein
the estimating module (144) further determines an exercise activity currently exercised by the user based on the collected data; and
the advice concluding module (146) further concludes an exercise advice based on the estimated current physical condition and the determined exercise activity of the user.

11. The scale according to any one of claims 8 to 10, wherein the advice concluding module (146) concludes the advice based on an environmental data around the user among the collected data.

12. The scale according to any one of claims 8 to 11, wherein the modules further comprise:
an identification recognizing module, receiving a plurality user information, wherein each user information comprising a recognized identification information of a corresponding user, and wherein the recognized identification information comprising at least one biometric feature of the corresponding user, measuring the at least one biometric feature of a first user by using a sensor for the at least one biometric feature disposed in the scale in response to detecting the first user steps on the scale (10), comparing the measured at least one biometric feature of the first user with the received recognized identification information and recognizing an identification of the first user at least based on the at least one biometric feature, determining whether the data of first user measured by the scale comprises a body fat percentage data according to the received user information, and activating the scale to measure data of the first user in response to the recognized identification and measuring the body fat percentage data of the first user in response to determining the date of the first user comprises the body fat percentage data.

13. The scale according to any one of claims 8 to 12, wherein the estimating module further weights the collected data according to an exercise activity type of the user and estimates a TDEE data of the user based on the weighted data, wherein the exercise activity type is determined based on a plurality of data previously collected from the sensing devices and recorded in a history of the user; and
the advice concluding module further concludes the advice based on the TDEE data.

## Patentansprüche

1. Verfahren zur Verwendung einer elektronischen Waage (10), um über einen physischen Zustand zu informieren, wobei das Verfahren die folgenden Schritte aufweist, die durch einen Prozessor (20) der Waage (10) ausgeführt werden:
Konfigurieren (S202, S402) einer Vielzahl von Abfühlvorrichtungen (12, 22, 32, 44) für einen Nutzer, um die Daten bezüglich des physischen Zustands des Nutzers zu messen;
Sammeln (S204, S404) der Daten, die durch die Abfühlvorrichtungen (12, 22, 32, 44) gemessen werden;
Integrieren bzw. Zusammenfügen (S206, S408), der gesammelten Daten, um einen gegenwärtigen physischen bzw. physischen Zustand des Nutzers zu schätzen;
Zusammenstellen (S208, S408) einer Empfehlung zumindest basierend auf dem aktuellen physischen Zustand des Nutzers; und
Anzeigen (S210, S410) der Empfehlung;
**dadurch gekennzeichnet, dass** der Schritt des Konfigurierens (S202, S402) Folgendes aufweist:
Detektieren (S802) einer Last, die auf die Waage (10) einwirkt;
Bestimmen (S804), ob die Last durch ein einzelnes Objekt oder zwei Objekte vorgesehen wird, und zwar gemäß einer Verschiebung in einem Schwerpunkt der Last und einer Veränderung eines Gewichts, das durch die Waage (10) gemessen wird;
Bestimmen (S808), ob die Last über einen vorbestimmten Zeitraum besteht, und zwar ansprechend darauf, dass bestimmt wird, dass die Last durch das einzelne Objekt vorgesehen wird;
Auslösen, dass die Waage in einen Menümodus eintritt (S810), und zwar ansprechend darauf, dass die Last innerhalb der vorbestimmten Zeit andauert; und
Auslösen, dass die Waage das Messen (S806) des Gewichts des Nutzers fortsetzt, und zwar ansprechend darauf, dass bestimmt wird, dass die Last durch zwei Objekte vorgesehen wird, oder die Last über die vorbestimmte Zeit andauert.

2. Verfahren gemäß Anspruch 1, wobei nach dem Schritt (S404) des Sammelns von Daten, die durch jede der Abfühlvorrichtungen (32, 44) gemessen werden, das Verfahren Folgendes aufweist:
Hochladen (S406) der gesammelten Daten auf einen Server (46), einschließlich Daten, die von den anderen Nutzern gesammelt wurden, um die gesammelten Daten mit den Daten von den anderen Nutzern zu vergleichen, und zwar gemäß einer Nutzerinformation des Nutzers und um die Empfehlung gemäß den gesammelten Daten von den anderen Nutzern und der Nutzerinformation des Nutzers festzulegen; und
Empfangen (S410) der Empfehlung von dem Server (46) zur Anzeige.

3. Verfahren gemäß Anspruch 1 oder 2, wobei nach dem Schritt (S204, S404) des Sammelns von Daten, die durch die Abfühlvorrichtungen gemessen werden, das Verfahren ferner Folgendes aufweist:
Bestimmen (S502) einer Übungs- bzw. Trainingsaktivität, die gegenwärtig durch den Nutzer ausgeübt wird, und zwar basierend auf den gesammelten Daten; und
Festlegen (S504) einer Trainingsempfehlung basierend auf dem geschätzten aktuellen physischen Zustand und der bestimmten Trainingsaktivität des Nutzers.

4. Verfahren gemäß Anspruch 1 bis 3, wobei der Schritt (S208, S408, S504) des Festlegens der Empfehlung zumindest auf dem aktuellen physischen Zustand des Nutzers ferner Folgendes aufweist:
Festlegen der Empfehlung basierend auf Umweltdaten um den Nutzer herum.

5. Verfahren gemäß einem der Ansprüche 1 bis 4, wobei die Abfühlvorrichtungen (12, 22, 32, 44) eine Waage aufweisen und der Schritt (S202, S402) des Konfigurierens der Vielzahl von Abfühlvorrichtungen (12, 22, 32, 44) für den Nutzer, um die Daten des Nutzers zu messen, Folgendes aufweist:
Empfangen einer Vielzahl von Nutzerinformationen, wobei jede der Nutzerinformationen eine erkannte Identifikationsinformation eines entsprechenden Nutzers aufweist, und wobei die erkannte Identifikationsinformation zumindest ein biometrisches Merkmal des entsprechenden Nutzers aufweist;
Detektieren, dass ein erster Nutzer auf die Waage steigt;
ansprechend darauf, dass detektiert wird, dass der erste Nutzer auf die Waage steigt, Messen des zumindest einen biometrischen Merkmals des ersten Nutzers durch Verwenden eines Sensors für das zumindest eine biometrische Merkmal bei der Waage;
Vergleichen des zumindest einen biometrischen Merkmals des ersten Nutzers mit der empfangenen, erkannten Identifikationsinformation und Erkennen der Identifikation des ersten Nutzers zumindest basierend auf dem zumindest einen biometrischen Merkmal;
Bestimmen, ob die Daten des ersten Nutzers, die durch die Waage gemessen werden, Daten zu einem Körperfettanteil gemäß der empfangenen Nutzerinformation aufweisen; und
Aktivieren der Waage, um Daten des ersten Nutzers zu messen, und zwar ansprechend auf die erkannte Identifikation und Messen des Körperfettanteils des ersten Nutzers ansprechend darauf, dass bestimmt wird, dass die Daten des ersten Nutzers die Körperfettanteilsdaten aufweisen.

6. Verfahren gemäß einem der Ansprüche 1 bis 5, wobei die Schritte (S206, S408) des Integrierens der gesammelten Daten, um den gegenwärtigen physischen Zustand des Nutzers zu schätzen, und des Festlegens (S208, S408) der Empfehlung Folgendes aufweisen:
Gewichten der gesammelten Daten gemäß eines Trainingsaktivitätstyps des Nutzers, wobei der Trainingsaktivitätstyp basierend auf einer Vielzahl von Daten bestimmt wird, die zuvor von den Abfühlvorrichtungen gesammelt und in einer Historie bzw. einem Verlauf des Nutzers aufgezeichnet wurden; und Schätzen von Daten des Tagesgesamtenergieaufwandes bzw. TDEE (TDEE = Total Daily Energy Expenditure) des Nutzers basierend auf den gewichteten Daten und Festlegen der Empfehlung basierend auf den TDEE-Daten.

7. Nicht-flüchtiges, computerlesbares Medium, das Anweisungen aufweist, eine Waage zu veranlassen, das Verfahren gemäß einem der Ansprüche 1 bis 6 auszuführen.

8. Waage (10) zur Mitteilung eines physischen Zustands, wobei die Waage (10) Folgendes aufweist:
eine Verbindungseinrichtung (18, 38), die sich mit einer Vielzahl von Abfühlvorrichtungen (22, 44) verbindet, die durch einen Nutzer getragen oder von diesem konfiguriert werden, wobei die Abfühlvorrichtungen (22, 44) konfiguriert sind, um Daten zu messen, die sich auf den physischen Zustand des Nutzers beziehen;
eine Anzeigevorrichtung (16, 36);
eine Speichervorrichtung (14, 34), die konfiguriert ist, um eine Vielzahl von Modulen (142, 144, 146, 148; 342, 344, 346) aufzuzeichnen; und
einen Prozessor (20, 42), der mit der Verbindungsvorrichtung (18, 38), der Speichervorrichtung (14, 34) und der Anzeigevorrichtung (16, 36) gekoppelt und konfiguriert ist, um auf die Module (142, 144, 146, 148; 342, 344, 346), die in der Speichervorrichtung (14, 34) aufgezeichnet sind, zuzugreifen und diese auszuführen, wobei die Module (142, 144, 146, 148; 342, 344, 346) Folgendes aufweisen:
ein Datensammelmodul (142, 342), das die Daten sammelt, die sich auf den physischen Zustand des Nutzers beziehen, die durch die Abfühlvorrichtungen (22, 44) gemessen werden;
ein Schätzmodul (144), das die gesammelten Daten integriert, um einen aktuellen physischen Zustand des Nutzers zu schätzen;
ein Empfehlungszusammenstellungsmodul (146), das eine Empfehlung basierend auf dem aktuellen physischen Zustand des Nutzers zusammenstellt;
ein Anzeigemodul (148), das die Empfehlung durch die Anzeigevorrichtung (16, 36) anzeigt;
und **gekennzeichnet durch**
ein Objektdetektionsmodul, das eine Last detektiert, die auf die Waage (10) einwirkt, das bestimmt, dass die Last durch ein einzelnes Objekt oder durch zwei Objekte vorgesehen wird, und zwar gemäß einer Verschiebung des Schwerpunkts und einer Veränderung eines Gewichts, das durch die Waage (10) gemessen wird, wobei bestimmt wird, ob die Last über eine vorbestimmte Zeit hinweg besteht, und zwar ansprechend darauf, dass bestimmt wird, dass die Last durch das einzelne Objekt vorgesehen wird, wobei ausgelöst wird, dass die Waage (10) in einen Menümodus eintritt, und zwar ansprechend darauf, dass die Last innerhalb der vorbestimmten Zeit besteht, und wobei ausgelöst wird, dass das Messen des Gewichts des Nutzers fortgesetzt wird, und zwar ansprechend darauf dass bestimmt wird, dass die Last durch zwei Füße vorgesehen wird oder die Last über die vorbestimmte Zeit hinweg besteht.

9. Waage gemäß Anspruch 8, die ferner Folgendes aufweist:
eine Kommunikationseinrichtung (40), die konfiguriert ist, um sich mit einem Netzwerk zu verbinden, und die Module ferner ein Kommunikationsmodul (344) zum Hochladen der gesammelten Daten auf einen Server (46) aufweisen, einschließlich der Daten, die von den anderen Nutzern durch das Netzwerk gesammelt werden, um die gesammelten Daten des Nutzers zu integrieren, die gesammelten Daten mit den Daten von den anderen Nutzern gemäß einer Nutzerinformation des Nutzers zu vergleichen und um die Empfehlung gemäß den gesammelten Daten von den anderen Nutzern und der Nutzerinformation des Nutzers zusammenzustellen, und um die Empfehlung von dem Server (46) zu empfangen.

10. Waage gemäß Anspruch 8 oder 9, wobei
das Schätzmodul (144) ferner eine Trainingsaktivität bestimmt, die gegenwärtig durch den Nutzer ausgeübt wird, und zwar basierend auf den gesammelten Daten; und
das Empfehlungszusammenstellungsmodul (146) ferner eine Trainingsempfehlung basierend auf dem geschätzten, aktuellen, physischen Zustand und der bestimmten Trainingsaktivität des Nutzers zusammenstellt.

11. Waage gemäß einem der Ansprüche 8 bis 10, wobei das Empfehlungszusammenstellungsmodul (146) die Empfehlung basierend auf Umweltdaten um den Nutzer herum innerhalb der gesammelten Daten zusammenstellt.

12. Waage gemäß einem der Ansprüche 8 bis 11, wobei die Module ferner Folgendes aufweisen:
ein Identifikationserkennungsmodul, das eine Vielzahl von Nutzerinformationen empfängt, wobei jede Nutzerinformation eine erkannte Identifikationsinformation eines entsprechenden Nutzers aufweist, und wobei die erkannte Identifikationsinformation zumindest ein biometrisches Merkmal des entsprechenden Nutzers aufweist, das zumindest eine biometrische Merkmal des ersten Nutzers unter Verwendung eines Sensors für das zumindest eine biometrische Merkmal, der an der Waage angeordnet ist, misst, und zwar ansprechend auf das Detektieren, dass der erste Nutzer auf die Waage (10) steigt, das gemessene zumindest eine biometrische Merkmal des ersten Nutzers mit der empfangenen, erkannten Identifikationsinformation vergleicht und eine Identifizierung des ersten Nutzers basierend auf dem zumindest einen biometrischen Merkmal erkennt, bestimmt, ob die Daten des ersten Nutzers, die durch die Waage gemessen werden, Daten zum Körperfettanteil gemäß der empfangen Nutzerinformation aufweisen, und die Waage aktiviert, die Daten des ersten Nutzers ansprechend auf die erkannte Identifizierung zu messen, und die Daten zum Körperfettanteil des ersten Nutzers misst, und zwar ansprechend auf die Bestimmung, dass die Daten des ersten Nutzers die Daten zum Körperfettanteil aufweisen.

13. Waage gemäß einem der Ansprüche 8 bis 12, wobei
das Schätzmodul ferner die gesammelten Daten gemäß eines Trainingsaktivitätstyps des Nutzers gewichtet und TDEE-Daten des Nutzers basierend auf den gewichteten Daten schätzt, wobei der Trainingsaktivitätstyp basierend auf einer Vielzahl von Daten bestimmt wird, die zuvor von den Abfühlvorrichtungen gesammelt und in einem Verlauf des Nutzers aufgezeichnet wurden; und
das Empfehlungszusammenstellungsmodul die Empfehlung basierend auf den TDEE-Daten zusammenstellt.

## Revendications

1. Procédé d'utilisation d'une balance électronique (10) pour aviser d'une condition physique, le procédé comprenant les étapes suivantes mises en oeuvre par un processeur (20) de la balance (10) :
la configuration (S202, S402) d'une pluralité de dispositifs de détection (12, 22, 32, 44) pour un utilisateur pour mesurer les données relatives à la condition physique de l'utilisateur ;
la collecte (S204, S404) des données mesurées par les dispositifs de détection (12, 22, 32, 44) ;
l'intégration (S206, S408) des données collectées pour estimer une condition physique courante de l'utilisateur ;
la conclusion (S208, S408) d'un avis au moins sur la base de la condition physique courante de l'utilisateur ; et
l'annonce (S210, S410) de l'avis ;
**caractérisé en ce que** l'étape de configuration (S202, S402) comprend :
la détection (S802) d'une charge agissant sur la balance (10) ;
la détermination (S804) de si la charge est assurée par un seul objet ou par deux objets en fonction d'un décalage sur un centre de gravité de la charge et d'un changement de poids mesuré par la balance (10) ;
la détermination (S808) de si la charge dure pendant une durée prédéterminée en réponse au fait que la charge est déterminée comme étant assurée par un seul objet ;
la commande de la balance pour entrer (S810) dans un mode de menu en réponse au fait que la charge dure le temps prédéterminé ; et
la commande de la balance pour continuer à mesurer (S806) le poids de l'utilisateur en réponse au fait que la charge est déterminée comme étant assurée par deux objets ou que la charge dure la durée prédéterminée.

2. Procédé selon la revendication 1, dans lequel, après l'étape (S404) de collecte des données mesurées par chacun des dispositifs de détection (32, 44), le procédé comprend en outre :
le téléchargement (S406) des données collectées sur un serveur (46) comprenant des données collectées à partir d'autres utilisateurs pour comparer les données collectées aux données des autres utilisateurs en fonction d'informations utilisateur de l'utilisateur, et pour conclure l'avis en fonction des données collectées des autres utilisateurs et des informations utilisateur de l'utilisateur ; et
la réception (S410) de l'avis provenant du serveur (46) pour l'annoncer.

3. Procédé selon la revendication 1 ou 2, dans lequel, après l'étape (S204, S404) de collecte des données mesurées par les dispositifs de détection, le procédé comprend en outre les étapes suivantes :
la détermination (S502) d'une activité d'exercice en cours d'exécution par l'utilisateur sur la base des données collectées ; et
la conclusion (S504) d'un avis d'exercice sur la base de la condition physique courante estimée et de l'activité d'exercice déterminée de l'utilisateur.

4. Procédé selon l'une quelconque des revendications 1 à 3, dans lequel l'étape (S208, S408, S504) de conclusion de l'avis au moins sur la base de la condition physique courante de l'utilisateur comprend en outre :
la conclusion de l'avis sur la base de données environnementales autour de l'utilisateur.

5. Procédé selon l'une quelconque des revendications 1 à 4, dans lequel les dispositifs de détection (12, 22, 32, 44) comprennent une balance et l'étape (S202, S402) de configuration de la pluralité de dispositifs de détection (12, 22, 32, 44) pour que l'utilisateur mesure les données de l'utilisateur comprend les étapes suivantes :
la réception d'une pluralité d'informations utilisateur, chaque information utilisateur comprenant des informations d'identification reconnue d'un utilisateur correspondant, et les informations d'identification reconnue comprenant au moins une caractéristique biométrique de l'utilisateur correspondant ;
la détection qu'un premier utilisateur monte sur la balance ;
en réponse à la détection que le premier utilisateur monte sur la balance, la mesure de ladite au moins une caractéristique biométrique du premier utilisateur en utilisant un capteur pour ladite au moins une caractéristique biométrique dans la balance ;
la comparaison de ladite au moins une caractéristique biométrique mesurée du premier utilisateur avec les informations d'identification reconnue reçues et la reconnaissance d'une identification du premier utilisateur au moins sur la base de ladite au moins une caractéristique biométrique ;
la détermination de si les données du premier utilisateur mesurées par la balance comprennent des données de pourcentage de graisse corporelle en fonction des informations utilisateur reçues ; et
l'activation de la balance pour mesurer des données du premier utilisateur en réponse à l'identification reconnue et la mesure des données de pourcentage de graisse corporelle du premier utilisateur en réponse au fait que la détermination des données du premier utilisateur comprend les données de pourcentage de graisse corporelle.

6. Procédé selon l'une quelconque des revendications 1 à 5, dans lequel les étapes (S206, S408) d'intégration des données collectées pour estimer la condition physique courante de l'utilisateur et de conclusion (S208, S408) de l'avis comprennent les étapes suivantes :
la pondération des données collectées en fonction d'un type d'activité physique de l'utilisateur, le type d'activité physique étant déterminé sur la base d'une pluralité de données précédemment collectées par les dispositifs de détection et enregistrées dans un historique de l'utilisateur ; et
l'estimation de données de dépense énergétique quotidienne totale, TDEE, de l'utilisateur sur la base des données pondérées et la conclusion de l'avis sur la base des données TDEE.

7. Support lisible par ordinateur non transitoire comprenant des instructions pour forcer une balance à exécuter le procédé selon l'une quelconque des revendications 1 à 6.

8. Balance (10) pour informer d'une condition physique, la balance (10) comprenant :
un dispositif de connexion (18, 38), se connectant à une pluralité de dispositifs de détection (22, 44) portés par ou configurés pour un utilisateur, les dispositifs de détection (22, 44) étant configurés pour mesurer des données relatives à la condition physique de l'utilisateur ;
un périphérique d'annonce (16, 36) ;
un dispositif de stockage (14, 34), configuré pour enregistrer une pluralité de modules (142, 144, 146, 148 ; 342, 344, 346) ; et
un processeur (20, 42), couplé au dispositif de connexion (18, 38), au dispositif de stockage (14, 34) et au dispositif d'annonce (16, 36), et configuré pour accéder et exécuter les modules (142, 144, 146, 148; 342, 344, 346) enregistrés dans le dispositif de stockage (14, 34), les modules (142, 144, 146, 148 ; 342, 344, 346) comprenant :
un module de collecte de données (142, 342), collectant les données relatives à la condition physique de l'utilisateur mesurées par les dispositifs de détection (22, 44) ;
un module d'estimation (144), intégrant les données collectées pour estimer une condition physique courante de l'utilisateur ;
un module de conclusion d'avis (146), concluant un avis au moins sur la base de la condition physique courante de l'utilisateur ;
un module d'annonce (148), annonçant l'avis au moyen du périphérique d'annonce (16, 36) ; et **caractérisé par**
un module de détection d'objet, détectant une charge appliquée sur la balance (10), déterminant que la charge est exercée par un seul objet ou par deux objets en fonction d'un décalage du centre de gravité de la charge et d'un changement de poids mesuré par la balance (10), déterminant si la charge dure pendant une durée prédéterminée en réponse à ce que la charge est déterminée comme étant exercée par un seul objet, commandant à la balance (10) d'entrer dans un mode de menu en réponse au fait que la charge dure pendant la durée prédéterminée, et en commandant à la balance (10) de continuer à mesurer le poids de l'utilisateur en réponse au fait que la charge est déterminée comme étant exercée par deux pieds ou que la charge dure pendant la durée prédéterminée.

9. Balance selon la revendication 8, comprenant en outre :
un dispositif de communication (40), configuré pour se connecter à un réseau, et les modules comprennent en outre un module de communication (344) pour télécharger les données collectées sur un serveur (46) comprenant des données collectées provenant d'autres utilisateurs à travers le réseau pour intégrer les données collectées de l'utilisateur, pour comparer les données collectées aux données provenant des autres utilisateurs en fonction des informations utilisateur de l'utilisateur, et pour conclure l'avis en fonction des données collectées provenant des autres utilisateurs et des informations utilisateur de l'utilisateur, et en recevant l'avis depuis le serveur (46).

10. Balance selon la revendication 8 ou 9, dans laquelle
le module d'estimation (144) détermine en outre une activité d'exercice en cours d'exécution par l'utilisateur sur la base des données collectées ; et
le module de conclusion d'avis (146) conclut en outre un avis d'exercice sur la base de la condition physique courante estimée et de l'activité d'exercice déterminée de l'utilisateur.

11. Balance selon l'une quelconque des revendications 8 à 10, dans laquelle le module de conclusion d'avis (146) conclut l'avis sur la base de données environnementales autour de l'utilisateur parmi les données collectées.

12. Balance selon l'une quelconque des revendications 8 à 11, dans laquelle les modules comprennent en outre :
un module de reconnaissance d'identification, recevant une pluralité d'informations utilisateur, chaque information utilisateur comprenant des informations d'identification reconnue d'un utilisateur correspondant, et les informations d'identification reconnue comprenant au moins une caractéristique biométrique de l'utilisateur correspondant, mesurant ladite au moins une caractéristique biométrique d'un premier utilisateur en utilisant un capteur pour ladite au moins une caractéristique biométrique disposée dans la balance en réponse à la détection que le premier utilisateur monte sur la balance (10), comparant ladite au moins une caractéristique biométrique mesurée du premier utilisateur avec les informations d'identification reconnue reçues et reconnaissant une identification du premier utilisateur au moins sur la base de ladite au moins une caractéristique biométrique, déterminant si les données du premier utilisateur mesurées par la balance comprennent un pourcentage de graisse corporelle en fonction des informations utilisateur reçues et activant la balance pour mesurer des données du premier utilisateur en réponse à l'identification reconnue et pour mesurer des données de pourcentage de graisse corporelle du premier utilisateur en réponse à la détermination que les données du premier utilisateur comprennent les données de pourcentage de graisse corporelle.

13. Balance selon l'une quelconque des revendications 8 à 12, dans laquelle
le module d'estimation pondère en outre les données collectées en fonction d'un type d'activité physique de l'utilisateur et estime des données TDEE de l'utilisateur sur la base des données pondérées, le type d'activité physique étant déterminé sur la base d'une pluralité de données précédemment collectées par les dispositifs de détection et enregistrées dans un historique de l'utilisateur ; et
le module de conclusion d'avis conclut en outre les avis sur la base des données TDEE.
